(19) **Europäisches Patentamt / European Patent Office / Office européen des brevets**

(11) **EP 4 737 949 A1**

(12) **EUROPEAN PATENT APPLICATION**
published in accordance with Art. 153(4) EPC

(43) Date of publication:
06.05.2026 Bulletin 2026/19

(21) Application number: 24910529.7

(22) Date of filing: 29.11.2024

(51) International Patent Classification (IPC):
*G01T 1/161* (2006.01)    *G06T 11/00* (2026.01)

(86) International application number:
PCT/CN2024/135750

(87) International publication number:
WO 2025/139596 (03.07.2025 Gazette 2025/27)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 30.12.2023 CN 202311859527

(71) Applicant: RAYSOLUTION Healthcare Co., Ltd
Hefei, Anhui 230093 (CN)

(72) Inventors:
• LI, Ang
  Hefei, Anhui 230093 (CN)
• FANG, Lei
  Hefei, Anhui 230093 (CN)
• LI, Bingxuan
  Hefei, Anhui 230093 (CN)
• ZHANG, Bo
  Hefei, Anhui 230093 (CN)

(74) Representative: FDST Patentanwälte
Nordostpark 16
90411 Nürnberg (DE)

(54) **IMAGE RECONSTRUCTION METHOD AND SYSTEM, DETECTION DEVICE, ELECTRONIC DEVICE AND STORAGE MEDIUM**

(57) The application discloses an image reconstruction method, a system, a detection device, an electronic device, and a storage medium, relating to the field of data processing. The image reconstruction method comprises: obtaining TOF resolutions of a portion of lines of response according to prior information; obtaining timing resolutions of all scintillation crystals according to the TOF resolutions of the portion of lines of response and a predetermined model; obtaining TOF resolutions of remaining lines of response according to the timing resolutions of all scintillation crystals and the predetermined model; and reconstructing an image according to all TOF resolutions and a first predetermined algorithm. The solution of the application applies differentiated TOF resolutions to different lines of response. In particular, by correlating the TOF resolutions of lines of response with the timing resolutions of corresponding scintillation crystals, the TOF resolutions of lines of response may be accurately estimated, thereby effectively enhancing image reconstruction quality .

```
┌─────────────────────────────────────────────────────────┐
│ Obtaining TOF resolutions of a portion of lines of      │ ⌐ S110
│ response according to prior information                 │
└─────────────────────────────────────────────────────────┘
                          │
                          ▼
┌─────────────────────────────────────────────────────────┐
│ Obtaining timing resolutions of all scintillation       │ ⌐ S120
│ crystals according to the TOF resolutions of the        │
│ portion of lines of response and a predetermined model  │
└─────────────────────────────────────────────────────────┘
                          │
                          ▼
┌─────────────────────────────────────────────────────────┐
│ Obtaining TOF resolutions of the remaining lines of     │ ⌐ S130
│ response according to the timing resolutions of all     │
│ scintillation crystals and the predetermined model      │
└─────────────────────────────────────────────────────────┘
                          │
                          ▼
┌─────────────────────────────────────────────────────────┐
│ Reconstructing an image according to all TOF            │ ⌐ S140
│ resolutions and a first predetermined algorithm         │
└─────────────────────────────────────────────────────────┘
```

FIG. 1

## Description

The application claims priority to Chinese Patent Application No. 202311859527.5, filed on December 30, 2023, the entire contents of which are incorporated herein by reference.

## Technical Field

The application relates to the field of image reconstruction, and specifically relates to an image reconstruction method, a system, a detection device, an electronic device, and a storage medium.

## Background

Positron Emission Tomography (PET) is one of the most advanced molecular imaging technologies in the world today, with numerous functions such as early cancer detection, therapeutic efficacy evaluation, pharmacokinetics observation and the like. By imaging compounds labeled with radionuclides in living organisms, PET is capable of non-invasively, quantitatively, and dynamically evaluating the metabolic level, biochemical reaction, and functional activity of various functional organs in living organisms. With high sensitivity and accuracy, PET has an irreplaceable position in clinical medicine and drug development.

At the beginning of development of PET, image reconstruction mainly relies on information regarding the number of coincidence events on the connecting line between detectors. However, early detectors had insufficient photon time of flight (Time of Flight, TOF) resolution performance, which may hardly bring any gain to image reconstruction. But with the development of PET detector technology in recent years, the TOF resolution of PET detectors has reached 200ps-600ps. With the support of such TOF information, the quality of TOF image reconstruction has been significantly improved compared to non-TOF image reconstruction. TOF resolution has also become a key metric for evaluating PET system performance, and meanwhile TOF image reconstruction has been widely used.

In existing methods, the coincidence events detected along a line of response (LOR) are sorted into discrete time bins, with each time bin representing a corresponding spatial segment of the LOR. TOF image reconstruction requires modeling the contribution $H_{ijk}$ of gamma-ray photon emitted from the $j$-th voxel to the $k$-th time bin on the $i$-th LOR, where $H_{ijk} = H_{ij}K_{ijk}$, which $K_{ijk}$ is commonly referred to as the TOF kernel. Typically, a Gaussian distribution is used to model the value of the TOF kernel in the $k$-th time bin, with the full width at half maximum (FWHM) of the Gaussian distribution being the TOF resolution of the PET system. It is generally assumed that all LORs have the same TOF resolution. Under this assumption, the TOF kernel of any LOR is modeled with the same standard deviation of the Gaussian distribution (i.e., the FWHM of the Gaussian distribution divided by 2.355). However, in actual systems, the TOF resolution differs among individual LORs. These differences distort the modeling of TOF information and therefore prevent TOF image reconstruction from achieving optimal performance.

The content described in the background is provided solely to facilitate understanding of the related technologies in this field and shall not be construed as an admission of prior art.

## Summary

Therefore, the application intends to provide an image reconstruction method and system, a detection device, an electronic device, and a storage medium, which may solve at least one problem existing in the prior art.

In a first aspect, an image reconstruction method is provided, comprising: obtaining TOF resolutions of a portion of lines of response according to prior information; obtaining timing resolutions of all scintillation crystals according to the TOF resolutions of the portion of lines of response and a predetermined model; obtaining TOF resolutions of remaining lines of response according to the timing resolutions of all scintillation crystals and the predetermined model; and reconstructing an image according to all TOF resolutions and a first predetermined algorithm.

In an embodiment of the application, the obtaining timing resolutions of all scintillation crystals according to the TOF resolutions of the portion of lines of response and a predetermined model comprises: inputting the TOF resolutions of the portion of lines of response as input values respectively into the predetermined model to obtain the timing resolutions of all scintillation crystals; and the obtaining TOF resolutions of the remaining lines of response according to the timing resolutions of all scintillation crystals and the predetermined model comprises: grouping the timing resolutions corresponding to two scintillation crystals located on the same line of response as one group, and inputting all groups of timing resolutions as input values respectively into the predetermined model to obtain the TOF resolutions of the remaining lines of response.

In an embodiment of the application, the predetermined model is:

$$\sigma_{ij}^2 = \sigma_i^2 + \sigma_j^2$$

where $\sigma_i$ represents the timing resolution of scintillation crystal i, $\sigma_j$ represents the timing resolution of scintillation crystal j, and $\sigma_{ij}$ represents the TOF resolution of the line of response formed by scintillation crystal i and scintillation crystal j.

In an embodiment of the application, the predetermined model is:

$$\sigma_{ij}^2 = \sigma_i^2 + \sigma_i^2 + \delta_r$$

where $\sigma_i$ represents the timing resolution of scintillation crystal i, $\sigma_j$ represents the timing resolution of scintillation crystal j, $\delta_r$ represents a variable related to photon incident angle, and $\sigma_{ij}$ represents the TOF resolution of the line of response formed by scintillation crystal i and scintillation crystal j.

In an embodiment of the application, the prior information is obtained according to the following steps: conducting an image detection test under test conditions to obtain the number of coincidence events on all lines of response and the time difference of each coincidence event; selecting lines of response where the number of coincidence events reaches a first predetermined threshold as target lines of response; and obtaining the TOF resolutions of all the target lines of response as the prior information according to a second predetermined algorithm and the time difference of each coincidence event.

In an embodiment of the application, the second predetermined algorithm is:

$$\sigma = \sqrt{\frac{\sum_{i=1}^{n}(\Delta t_i - \overline{\Delta t})^2}{n-1}}$$

where n is the total number of coincidence events on a certain target line of response, $\Delta t_i$ is the time difference of the i-th coincidence event, $\overline{\Delta t}$ is the mean value of the coincidence time differences on the target line of response, $\sigma$ is the standard deviation of the time differences on the target line of response, and the TOF resolution of the target line of response is $2.355\sigma$.

In an embodiment of the application, the first predetermined threshold is greater than or equal to 5.

In an embodiment of the application, the test conditions comprise: a radioactive source that only emits positrons being selected as a target source; and/or, the shape of the target source comprising a shell source, a line source, or a point source.

In an embodiment of the application, when the target source is the shell source, before selecting lines of response where the number of coincidence events exceeds the first predetermined threshold as target lines of response, the steps for obtaining the prior information further comprise: excluding lines of response at a distance from the central axis of the shell source that reaches a second predetermined threshold.

In an embodiment of the application, the second predetermined threshold is 80% of the maximum distance corresponding to the field of view of the detector ring.

In an embodiment of the application, when the target source is the shell source, after selecting lines of response where the number of coincidence events exceeds the first predetermined threshold as target lines of response, the steps for obtaining the prior information further comprise: performing time-of-flight compensation on all coincidence events using a predetermined method.

In an embodiment of the application, the predetermined method comprises: obtaining two intersection points between the line of response and the shell source and obtaining the time-of-flight difference of coincidence events generated by annihilation at the two intersection points; plotting a time difference spectrum according to the time differences of the coincidence events to obtain two peaks, where the two peaks respectively correspond to the two intersection points between the line of response and the shell source; classifying the coincidence events into one of the two peaks using a k-means algorithm; and compensating the time-of-flight difference of the coincidence events according to the intersection point to which the coincidence event belongs and the calculated time-of-flight difference.

In an embodiment of the application, the test conditions further comprise: the size of the target source being less than a predetermined requirement.

In an embodiment of the application, the predetermined requirement comprises: the thickness of the shell source being less than or equal to 1 cm; or, the diameter of the line source being less than or equal to 1 cm; or, the diameter of the point source is less than or equal to 0.5 cm.

In an embodiment of the application, the test conditions further comprise: the position of the target source ensures that the connecting lines between any single detection unit and at least two detection units at different positions all pass through the target source.

In an embodiment of the application, the height extent of the shell source is parallel to the axis of the PET; or, among the intersection points between the cross-section of the detector ring and the line source, at least three points are not collinear; or, at least four of the point sources are not coplanar.

In an embodiment of the application, the target source comprises Fluorine-18 ($^{18}$F), Sodium-22 ($^{22}$Na), or Germanium-68 ($^{63}$Ge).

In an embodiment of the application, the first predetermined algorithm is:

$$x_j^{n+1} = \frac{x_j^n}{\sum_i H_{ij} N_i A_i} \sum_{(i,k) \in S} H_{ijk} \frac{1}{\sum_j H_{ijk} x_j^n + \frac{r_{ik} + s_{ik}}{N_i A_i}}$$

where $x_j^n$ is the j-th pixel value of the n-th iteration, $N_i$ and $A_i$ are respectively the normalization factor and attenuation factor of the i-th line of response, $r_{ik}$ and $s_{ik}$ are respectively the number of random and scatter coincidence events on the k-th time bin of the i-th line of response, S is the set of line of response numbers and time bin numbers where all list-mode events are located, $H_{ij}$ is the system matrix for non-TOF reconstruction, and $H_{ijk}$ is the system matrix for TOF reconstruction.

In an embodiment of the application, the first predetermined algorithm is:

$$x_j^{l+1,n} = \frac{x_j^{l,n}}{\sum_i H_{ij} N_i A_i} \sum_{(i,k) \in S_l} H_{ijk} \frac{1}{\sum_j H_{ijk} x_j^{l,n} + \frac{r_{ik} + s_{ik}}{N_i A_i}}$$

where $x_j^{l,n}$ is the j-th pixel value of the l-th subset of the n-th iteration, $N_i$ and $A_i$ are respectively the normalization factor and attenuation factor of the i-th line of response, $r_{ik}$ and $s_{ik}$ are respectively the number of random and scatter coincidence events on the k-th time bin of the i-th line of response, $S_l$ is the set of line of response number i and time bin number k where all list-mode events in the l-th subset are located, $H_{ij}$ is the system matrix for non-TOF reconstruction, and $H_{ijk}$ is the system matrix for TOF reconstruction.

In an embodiment of the application, the first predetermined algorithm is:

$$x_j^{n+1} = \frac{x_j^n}{\sum_i H_{ij} N_i A_i} \sum_i \sum_k H_{ijk} \frac{Y_{ik}}{\sum_j H_{ijk} x_j^n + \frac{r_{ik} + s_{ik}}{N_i A_i}}$$

where $x_j^n$ is the j-th pixel value of the n-th iteration, $N_i$ and $A_i$ are respectively the normalization factor and attenuation factor of the i-th line of response, $r_{ik}$ and $s_{ik}$ are respectively the number of random and scatter coincidence events on the k-th time bin of the i-th line of response, $H_{ij}$ is the system matrix for non-TOF reconstruction, and $H_{ijk}$ is the system matrix for TOF reconstruction.

In an embodiment of the application, the first predetermined algorithm is:

$$x_j^{l,+1n} = \frac{x_j^{l,n}}{\sum_i H_{ij} N_i A_i} \sum_{i \in S_l} \sum_k H_{ijk} \frac{Y_{ik}}{\sum_j H_{ijk} x_j^{l,n} + \frac{r_{ik} + s_{ik}}{N_i A_i}}$$

where $x_j^{l,n}$ is the j-th pixel value of the l-th subset of the n-th iteration, $N_i$ and $A_i$ are respectively the normalization factor and attenuation factor of the i-th line of response, $r_{ik}$ and $s_{ik}$ are respectively the number of random and scatter coincidence events on the k-th time bin of the i-th line of response, $S_l$ is the set of line of response number i in the l-th subset.

In an embodiment of the application,

$$H_{ijk} = H_{ij} K_{ijk}$$

$$K_{ijk} = \int_{T_0}^{T_1} \frac{1}{\sqrt{2\pi}\sigma} \exp\left(-\frac{t^2}{2\sigma^2}\right) dt$$

where T0 is the start time of the k-th time bin, T1 is the end time of the k-th time bin, t is an integration variable, and $\sigma$ represents the TOF resolution of the line of response.

In a second aspect, an image reconstruction system is provided, comprising: an acquisition unit, configured to obtain TOF resolutions of a portion of lines of response according to prior information; a calculation unit, configured to obtain timing resolutions of all scintillation crystals according to the TOF resolutions of the portion of lines of response and a predetermined model, and to obtain TOF resolutions of remaining lines of response according to the timing resolution of all scintillation crystals and the predetermined model; and a reconstruction unit, configured to reconstruct an image according to all TOF resolutions and a first predetermined algorithm.

In an embodiment of the application, the calculation unit is configured to input the TOF resolutions of the portion of lines of response as input values respectively into the predetermined model to obtain the timing resolution of all scintillation crystals, and to group the timing resolutions corresponding to two scintillation crystals located on the same line of response as one group, and input all groups of timing resolutions as input values respectively into the predetermined model to obtain the TOF resolutions of the remaining lines of response.

In a third aspect, a detection device is provided, comprising the image reconstruction system according to the second aspect.

In a fourth aspect, an electronic device is provided, comprising: a processor and a memory storing a computer program, wherein the processor is configured to execute the computer program to implement the method according to the embodiments of the application.

In a fifth aspect, a storage medium is provided, wherein the storage medium stores a computer program that, when executed, implements the method according to the embodiments of the application.

Compared with the prior art, the solution of the embodiments of the application performs differentiated processing on the TOF resolution of different lines of response, especially by associating the TOF resolution of lines of response with the timing resolution of corresponding scintillation crystals, and accurately estimating the TOF resolution of lines of response, which may effectively improve the quality of image reconstruction.

Some optional features and other effects of the embodiments of the application are described in part below and will become apparent in part through reading this document.

## Brief Description of Drawings

The application will be further explained by way of exemplary embodiments, which are described in detail with reference to the accompanying drawings. These embodiments are not limiting, and in these embodiments, the same reference numerals represent the like structures, wherein:

FIG. 1 shows a flowchart of an image reconstruction method according to an embodiment of the application;

FIG. 2 shows a flowchart of an image reconstruction method according to an embodiment of the application;

FIG. 3 shows a schematic cross-sectional view of a shell source and a detector according to an embodiment of the application;

FIG. 4 shows a schematic cross-sectional view of a line source/point source and a detector according to an embodiment of the application;

FIG. 5 shows an original time-difference distribution diagram on a line of response in a shell source according to an embodiment of the application;

FIG. 6 shows a TOF-difference-compensated distribution diagram on a line of response in a shell source according to an embodiment of the application;

FIG. 7 shows a simulated time-of-flight diagram for detection tests using a shell source according to an embodiment of the application;

FIG. 8 shows a block diagram of an image reconstruction system according to an embodiment of the application; and

FIG. 9 shows a schematic diagram of an electronic device according to an embodiment of the application.

## Detailed Description

In order to make the above objects, features, and advantages of the application more clearly understandable, the specific embodiments of the application will be described in detail below with reference to the accompanying drawings. Many specific details are set forth in the following description to facilitate a full understanding of the application. However, the application may be implemented in various ways different from those described herein, and those skilled in the art may make similar modifications without departing from the spirit of the application. Therefore, the application is not limited by the specific embodiments disclosed below.

It should be noted that when an element is referred to as being "fixed to" another element, it may be directly on the other element or there may also be an intervening element. When an element is considered to be "connected" to another element, it may be directly connected to the other element or there may be intervening elements present simultaneously. The terms "vertical", "horizontal", "left", "right", and similar expressions used herein are for illustrative purposes only. The described features, structures, or characteristics may be combined in one or more embodiments in any suitable manner. In the following description, numerous specific details are provided to give a thorough understanding of embodiments of the application. However, those skilled in the art will recognize that the technical solution of the application may be practiced without one or more of these specific details, or other methods, components, materials, devices, operations, etc. may be employed. In these cases, well-known structures, methods, devices, implementations, materials, or operations are not shown or described in detail.

The flowcharts shown in the drawings are merely exemplary illustrations and do not necessarily include all contents and operations/steps, nor have they to be performed in the described order. For example, some operations/steps may also be decomposed, and some operations/steps may be combined or partially combined, so the actual execution order may change according to actual situations.

The terms "first", "second", etc. in the specification and claims of the application and the above drawings are used to distinguish different objects, rather than to describe a specific order. Furthermore, the terms "including" and "having" and any variations thereof are intended to cover non-exclusive inclusions. For example, a process, method, system, product, or device that comprises a series of steps or units is not limited to the listed steps or units, but optionally also comprises steps or units not listed, or optionally also comprises other steps or units inherent to these processes, methods, products, or devices. The term "and/or" comprises any and all combinations of one or more of the associated listed items.

Unless otherwise defined, all technical and scientific terms used herein have the same meaning as commonly understood by those skilled in the art to which this application belongs. The terms used in the specification of the application are only for the purpose of describing specific embodiments and are not intended to limit the application.

The image reconstruction method, system, detection device, electronic device, and storage medium provided in the application particularly relate to Positron Emission Tomography (PET) technology.

As mentioned above, in the technology known to the inventors, existing TOF image reconstruction generally assumes that all lines of response (LORs) have the same TOF resolution. However, in actual systems, there are certain differences in the TOF resolution of each LOR. Such differences cause distortion in TOF information modeling, thereby preventing the achievement of optimal performance of TOF image reconstruction.

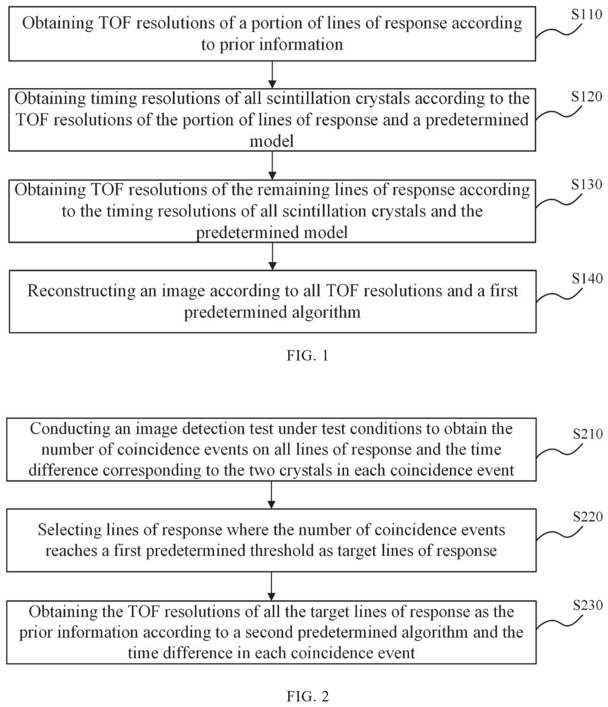

In this regard, the application provides an image reconstruction method. As shown in FIG. 1, the image reconstruction method provided by the application generally comprises the following steps:

S110: Obtaining TOF resolutions of a portion of lines of response according to prior information.

The requirement for the number or proportion of the portion of lines of response is that it is sufficient to obtain the timing resolutions of all scintillation crystals based on the TOF resolutions of the portion of lines of response. In practice, compared with existing TOF image reconstruction, where the TOF resolutions of all lines of response are assumed to be identical under extreme conditions, in the application, the TOF resolutions of all lines of response ultimately obtained based on the TOF resolutions of the portion of lines of response are generally not identical, which more closely reflects actual conditions.

It should be particularly noted that, generally speaking, a detection device comprises a plurality of detectors. By way of example, all detectors are combined to form at least one detector ring. When there are a plurality of detector rings, all the detector rings are concentrically stacked. Each detector comprises an array consisting of a plurality of detection units, and each detection unit comprises scintillation crystals and other devices and the like. When the connecting line between any two scintillation crystals passes through the field of view of the detector ring, it may constitute a line of response. That is, each end of a line of response is respectively connected to one scintillation crystal. In the application, it is required that the scintillation crystals connected to the two ends of the portion of lines of response need to cover all scintillation crystals. Expressed conversely, each scintillation crystal corresponds to at least one line of response.

The purpose of setting the number or proportion of the portion of lines of response in the application is to balance the amount of lines of response to be acquired and the computational load for TOF resolution calculation on the basis of ensuring that the basic data (TOF resolutions of the portion of lines of response) is sufficient to successfully obtain the TOF resolution of all lines of response. It may be understood that when the number of the portion of lines of response is large, the computational load for calculating the TOF resolutions of the remaining lines of response may be reduced. Otherwise, it will increase the computational load for calculating the TOF resolutions of the remaining lines of response. However, when the number of the portion of lines of response is large, it also requires that the data volume of the prior information be sufficiently large. Therefore, the number or proportion of the portion of lines of response should be appropriate.

S120: Obtaining timing resolutions of all scintillation crystals according to the TOF resolutions of the portion of lines of response and a predetermined model.

By way of example, step S120 may further comprise: inputting the TOF resolutions of the portion of lines of response as input values respectively into the predetermined model to obtain the timing resolutions of all scintillation crystals.

In the above step S110, the TOF resolutions of the portion of lines of response has been obtained according to the prior information, and because the number of the portion of lines of response is appropriate, the timing resolutions of all scintillation crystals may be obtained through the TOF resolutions of the portion of lines of response.

By way of example, the predetermined model is:

$$\sigma_{ij}^2 = \sigma_i^2 + \sigma_j^2 \qquad (1)$$

where $\sigma_i$ represents the timing resolution of scintillation crystal i, $\sigma_j$ represents the timing resolution of scintillation crystal j, and $\sigma_{ij}$ represents the TOF resolution of the line of response formed by scintillation crystal i and scintillation crystal j.

In actual operation, the timing resolutions of all scintillation crystals may be obtained by solving a system of simultaneous equations. For example, when $\sigma_{12}$, $\sigma_{13}$, $\sigma_{23}$... are known, by forming a system of simultaneous equations with the above formula (1), $\sigma_1$, $\sigma_2$, $\sigma_3$... may be obtained. By way of example, the solution of the system of simultaneous equations may adopt methods such as Preconditioned Conjugate Gradient (PCG), Minimum Residuals (MINRES), or Least Squares QR-factorization (LSQR). These methods may refer to the records of the prior art, and the application will not repeat them.

Those skilled in the art should understand that, in addition to the above formula (1), the predetermined model may also be other models or algorithms, as long as the model or algorithm that may obtain the TOF resolutions of all lines of response based on the TOF resolutions of the portion of lines of response may be applied to the application. For example, in a further example of the application, the above formula (1) may be extended and expanded. In practice, the inventors have found that the TOF resolution of a line of response is not only affected by scintillation crystals, but also by the photon incident angle. Therefore, on the basis of the above formula (1), a variable $\delta_r$ may be further introduced, where r is the distance value between the line of response and the center of the field of view (FOV) on the transaxial plane of the detector ring. The photon incident angle depends on r. The relationship between r and the variable $\delta_r$ may be obtained through experience, and the variable $\delta_r$ may be indexed through r. Specifically, the above formula (1) may be transformed to obtain the following formula (2):

$$\sigma_{ij}^2 = \sigma_i^2 + \sigma_j^2 + \delta_r \qquad (2)$$

where $\sigma_i$, $\sigma_j$, $\sigma_{ij}$ have the same meaning as in the above formula (1), and $\delta_r$ represents a variable related to photon incident angle.

When obtaining the timing resolutions of scintillation crystals and the TOF resolutions of lines of response using the above formula (2) as the predetermined model, since it further associates the TOF resolutions of lines of response with the photon incident angle on the basis of associating the TOF resolutions of lines of response with scintillation crystals, it may obtain more accurate timing resolution of scintillation crystals than the above formula (1), thereby obtaining more accurate TOF resolutions of lines of response, which is more conducive to the performance of image reconstruction.

For the solution of the above formula (2), the timing resolutions of all scintillation crystals may also be obtained by solving a system of simultaneous equations. For example, when $\sigma_{ij}$, $\sigma_{ik}$, $\sigma_{jk}$... are known, by forming a system of simultaneous equations with the above formula (2), $\sigma_i$, $\sigma_j$, $\sigma_k$... may be obtained. By way of example, the solution of the system of simultaneous equations may adopt methods such as Preconditioned Conjugate Gradient (PCG), Minimum Residuals (MINRES), or Least Squares QR-factorization (LSQR). These methods may refer to the records of the prior art, and the application will not repeat them.

S130: Obtaining TOF resolutions of remaining lines of response according to the timing resolutions of all scintillation crystals and the predetermined model.

It may be understood that the portion of lines of response in the above step S110 and the remaining lines of response in step S130 together constitute all lines of response.

By way of example, step S130 may further comprise: grouping the timing resolutions corresponding to two scintillation crystals located on the same line of response as one group, and inputting all groups of timing resolutions as input values respectively into the predetermined model to obtain the TOF resolution of the remaining lines of response.

By way of example, taking the predetermined model as the above formula (1) as an example, by performing permutation and combination of all scintillation crystals in pairs to form a plurality of groups, one group corresponds to one LOR, all groups correspond to all LORs, and each group is respectively substituted into the above formula (1) as an input value, the TOF resolutions of the remaining lines of response may be finally obtained.

It may be understood that the predetermined models used in step S120 and step S130 are generally the same, however, it is not excluded that the predetermined models used in the two steps may be different. For example, the predetermined model in step S120 may use formula (1), and the predetermined model in step S130 may use formula (2), or vice versa, or steps S120 and S130 respectively adopt other algorithms applicable to the present application as predetermined models.

S140: Reconstructing an image according to the TOF resolutions and a first predetermined algorithm.

The first predetermined algorithm may be any algorithm that may implement image reconstruction in the field of TOF image reconstruction.

In one example, the first predetermined algorithm is, for example, a list-mode maximum-likelihood expectation-maximization (LM-MLEM) algorithm, with the specific algorithm as follows in formula (3).

$$x_j^{n+1} = \frac{x_j^n}{\sum_i H_{ij} N_i A_i} \sum_{(i,k)\in S} H_{ijk} \frac{1}{\sum_j H_{ijk} x_j^n + \frac{r_{ik}+s_{ik}}{N_i A_i}} \qquad (3)$$

where $x_j^n$ is the j-th pixel value of the n-th iteration, $N_i$ and $A_i$ are respectively the normalization factor and attenuation factor of the i-th line of response, $r_{ik}$ and $s_{ik}$ are respectively the number of random and scatter coincidence events on the k-th time bin of the i-th line of response, S is the set of line of response numbers and time bin numbers where all list-mode events are located, $H_{ij}$ is the system matrix for non-TOF reconstruction, and $H_{ijk}$ as needed is the system matrix for TOF reconstruction.

In another example, the first predetermined algorithm is, for example, a list-mode ordered-subsets expectation-maximization (LM-OSEM) algorithm, with the specific algorithm as follows in formula (4):

$$x_j^{l+1,n} = \frac{x_j^{l,n}}{\sum_i H_{ij} N_i A_i} \sum_{(i,k)\in S_l} H_{ijk} \frac{1}{\sum_j H_{ijk} x_j^{l,n} + \frac{r_{ik}+s_{ik}}{N_i A_i}} \qquad (4)$$

where $x_j^{l,n}$ is the j-th pixel value of the l-th subset of the n-th iteration, $N_i$ and $A_i$ are respectively the normalization factor and attenuation factor of the i-th line of response, $r_{ik}$ and $s_{ik}$ are respectively the number of random and scatter coincidence events on the k-th time bin of the i-th line of response, $S_l$ is the set of line of response number i and time bin number k where all list-mode events in the l-th subset are located, $H_{ij}$ is the system matrix for non-TOF reconstruction, and $H_{ijk}$ is the system matrix for TOF reconstruction.

In another example, the first predetermined algorithm is, for example, a maximum-likelihood expectation-maximization (MLEM) algorithm, with the specific algorithm as follows in formula (5):

$$x_j^{n+1} = \frac{x_j^n}{\sum_i H_{ij} N_i A_i} \sum_i \sum_k H_{ijk} \frac{Y_{ik}}{\sum_j H_{ijk} x_j^n + \frac{r_{ik}+s_{ik}}{N_i A_i}} \qquad (5)$$

where $x_j^n$ is the j-th pixel value of the n-th iteration, $N_i$ and $A_i$ are respectively the normalization factor and attenuation factor of the i-th line of response, $r_{ik}$ and $s_{ik}$ are respectively the number of random and scatter coincidence events on the k-th time bin of the i-th line of response, $H_{ij}$ is the system matrix for non-TOF reconstruction, and $H_{ijk}$ is the system matrix for TOF reconstruction.

In another example, the first predetermined algorithm is, for example, an ordered-subsets expectation-maximization (OSEM) algorithm, with the specific algorithm as follows in formula (6):

$$x_j^{l,+1n} = \frac{x_j^{l,n}}{\sum_i H_{ij} N_i A_i} \sum_{i \in S_l} \sum_k H_{ijk} \frac{Y_{ik}}{\sum_j H_{ijk} x_j^{l,n} + \frac{r_{ik}+s_{ik}}{N_i A_i}} \qquad (6)$$

where $x_j^{l,n}$ is the j-th pixel value of the l-th subset of the n-th iteration, $N_i$ and $A_i$ are respectively the normalization factor and attenuation factor of the i-th line of response, $r_{ik}$ and $s_{ik}$ are respectively the number of random and scatter coincidence events on the k-th time bin of the i-th line of response, $S_l$ is the set of line of response number i in the l-th subset, $H_{ij}$ is the system matrix for non-TOF reconstruction, and $H_{ijk}$ is the system matrix for TOF reconstruction.

In the above examples, $H_{ijk} = H_{ij} K_{ijk}$, $K_{ijk}$ may be calculated using the following formula (7).

$$K_{ijk} = \int_{T_0}^{T_1} \frac{1}{\sqrt{2\pi}\sigma} \exp\left(-\frac{t^2}{2\sigma^2}\right) dt \qquad (7)$$

where T0 is the start time of the k-th time bin, T1 is the end time of the k-th time bin, t is an integration variable, and σ may adopt the data obtained in the predetermined model in the above step S130.

Compared with the prior art, the image reconstruction method provided by the application applies differentiated TOF resolutions to different lines of response. In particular, by correlating the TOF resolutions of lines of response with the timing resolutions of corresponding scintillation crystals, the TOF resolutions of lines of response may be accurately estimated and applied to TOF image reconstruction, thereby effectively enhancing image reconstruction quality.

Regarding prior information, it may be obtained in multiple ways in the application, for example, it may be obtained through simulation tests. By way of example, as shown in FIG. 2, the prior information is obtained according to the following steps:

S210: Conducting an image detection test under test conditions to obtain the number of coincidence events on all lines of response and the time difference corresponding to two crystals in each coincidence event.

Regarding test conditions, two aspects are mainly considered: one is the selection of detectors, and the other is the selection of radioactive sources.

Regarding the selection of detectors, it is generally required to use the same detectors as those used in actual image detection, including the shape and structure of the detectors, which are all required to be the same. In this way, it is possible to omit the step of correcting the prior information due to different detectors, thereby reducing the computational workload, while greatly ensuring the accuracy of the prior information.

Regarding the selection of radioactive sources, in the application, a radioactive source that only emits positrons is selected as the target source, that is, the target source is required not to emit gamma photons in the energy range of 400keV-650keV to avoid affecting the detection of positron annihilation. Examples of the target source include: Fluorine-18 ($^{18}$F), Sodium-22 ($^{22}$Na), or Germanium-68 ($^{68}$Ge).

In the application, the core considerations for the selection of the target source are the shape and the placement position in the detector.

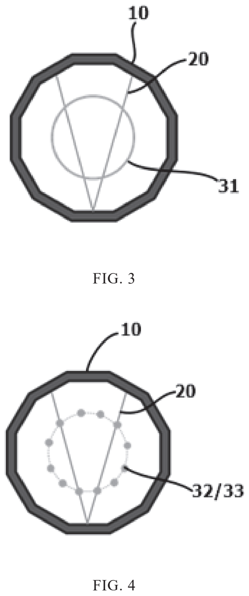

Regarding the shape, the shape of the target source(s) comprises a shell source, line sources, or point sources. A schematic cross-sectional view of the shell source is shown in FIG. 3, where the shell source 31 is placed within the detector ring 10, with the straight lines being lines of response 20. Generally, on the cross-section, there are at least two connecting lines between a single detection unit and two other detection units at different positions passing through the radioactive source. A schematic cross-sectional view of the line source(s) and point source(s) is shown in FIG. 4, where the points inside the detector ring 10 represent the cross-section of the line source(s) 32 or the point source(s) 33. The target source is required to be sufficiently thin, that is, the size of the target source(s) is required to be less than a predetermined requirement. For example, the thickness of the shell source is less than or equal to 1 cm, or the diameter(s) of the line source(s) is/are less than or equal to 1 cm, or the diameter(s) of the point source(s) is/are less than or equal to 0.5 cm. Regarding the shell source, those skilled in the art may understand that, as the name implies, the shell source has a shell, generally in the shape of a long cylinder, with an inner wall and an outer wall. The target source is placed between the inner wall and the outer wall. The above-mentioned thickness of the shell source represents the radial distance between the inner wall and the outer wall on the cross-section.

Regarding the placement position(s) of the target source(s) in the detector, the position(s) of the target source(s) is/are required to ensure that the connecting lines between any single detection unit and at least two detection units at different positions all pass through the target source(s), where the detector comprises a plurality of detection units, and each detection unit comprises scintillation crystals and photoelectric converters, etc. Specifically, when the target source is a shell source, the height extent of the shell source is parallel to the axis of the PET. When the target source(s) is/are line source(s), among the intersection points between the cross-section of the detector ring and the line source(s), at least three points are not collinear, which may be achieved by setting at least three line sources or by rotating the line source(s).

When the target source(s) is/are a point source(s), at least four points of the point source(s) are not coplanar, which may be achieved by setting at least four point sources or by rotating and/or moving the point source(s). In some examples, there is only one line source and one point source, and the purpose of having at least three non-collinear points at the intersection with the line source on the cross-section of the detector ring and having at least four non-coplanar point sources is achieved by rotating the line source and the point source.

In addition, the activity range of the shell source is 0.4 mCi-2 mCi, with a scan time in a range of 1 hour to 2 hours; the activity range of the line source(s) is 50μCi-1000μCi, with a scan time in a range of 5 minutes to 20 minutes for a single position; the activity range of the point source(s) is in a range of 10μCi to 200μCi, with a scan time in a range of 1 minute to 5 minutes for a single position.

In step S210, test conditions may be selected as described above to initiate the test. During the test, the number of coincidence events on all lines of response, the time differences corresponding to two crystals in single coincidence events, and other information are obtained and recorded. It should be particularly noted that a single coincidence event consists of two single photon events detected by the scintillation crystals corresponding to the two ends of the same line of response, and for each coincidence event, a photon detection time difference corresponding to the single photon events at the two ends of the line of response may be obtained.

S220: Selecting lines of response where the number of coincidence events reaches a first predetermined threshold as target lines of response.

During detection, some lines of response have a small number of coincidence events , and some lines of response even detect no coincidence events. Due to the small amount of data, subsequent information acquisition may be infeasible. Therefore, it is needed to select target lines of response based on the number of coincidence events. By way of example, the first predetermined threshold is greater than or equal to 5, that is, lines of response with more than 5 coincidence events are selected as target lines of response.

When the target source comprises point source(s) or line source(s), the TOF resolution of the line of response may be calculated directly. However, when the target source is a shell source, there is a problem of being unable to distinguish from which intersection point between the shell source and the line of response the coincidence event originates. Therefore, before step S220, the step of obtaining prior information also comprises:

Excluding lines of response at a distance from the central axis of the shell source that reaches a second predetermined threshold.

The second predetermined threshold is 80% of the maximum distance corresponding to the field of view of the detector ring. That is, lines of response at a distance from the central axis of the shell source that exceeds 80% of the maximum distance corresponding to the field of view of the detector ring are not target lines of response.

In addition, when the target source is a shell source, after step S220, the step of obtaining prior information further comprise:

Performing time-of-flight compensation on all coincidence events using a predetermined method.

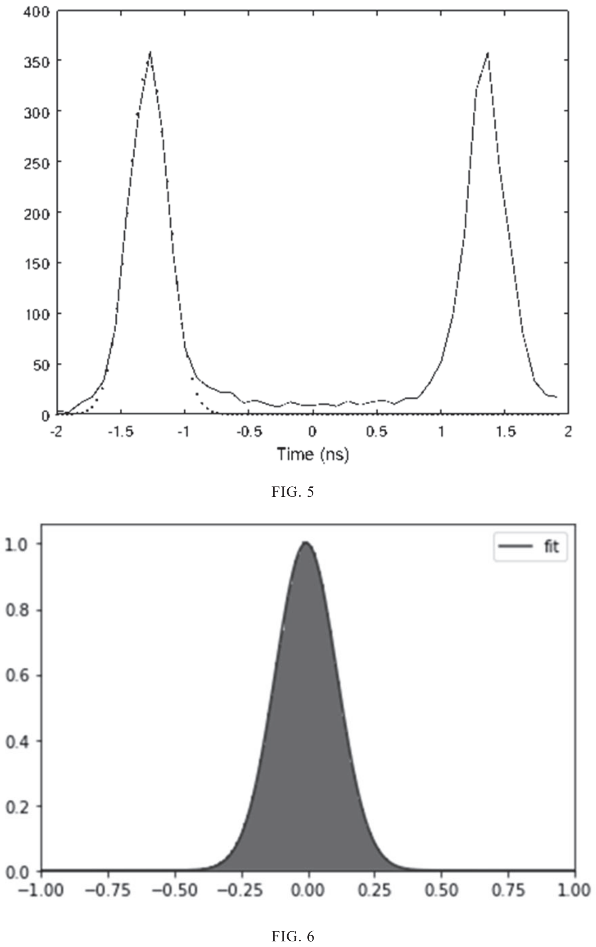

Performing time-of-flight compensation on coincidence events may eliminate the influence of time of flight on time difference. The predetermined method may be an existing time compensation method. In one example, the time-of-flight difference caused by two annihilation positions is calculated according to the geometric relationship, and then this time difference is used to compensate the coincidence events. As shown in FIG. 5, the distribution of time differences on a line of response in the shell source originally has two peaks. As shown in FIG. 6, after compensation, it becomes a single peak.

Specifically, in one example of the application, the predetermined method comprises:

Obtaining two intersection points between the line of response and the shell source and obtaining the time-of-flight difference of coincidence events generated by annihilation at the two intersection points;

Plotting a time difference spectrum according to the time differences of the coincidence events to obtain two peaks, where the two peaks respectively correspond to the two intersection points between the line of response and the shell source;

Classifying the coincidence events into one of the two peaks using a k-means algorithm; and

Compensating the time-of-flight difference of the coincidence events according to the intersection point to which the coincidence event belongs and the calculated time-of-flight difference.

More specifically, in one example, for a specific LOR, there are two intersection points with the shell source. The time-of-flight differences of coincidence events generated by annihilation at these two intersection points differ greatly, such that two "peaks" may be seen on the time difference spectrum, as shown in for example, FIG. 5. The time of flight of each coincidence event is compensated through the following method:

1. Assuming that N coincidence events are detected on this LOR, and the time-of-flight differences of each coincidence event are $\Delta t_i$, i = 1,2, ..., N, and calculating the center positions of the two peaks through the k-means algorithm:

    1.1) Initializing the center positions of the two peaks $\mu_1$ and $\mu_2$ to -1 and 1;

    1.2 For i = 1, 2, ..., N:

    1.2.1) If $|\Delta t_i - T_1| \leq |\Delta t_i - T_2|$, then the i-th coincidence event belongs to the first set $S_1$;

    1.2.2) If $|\Delta t_i - T_1| > |\Delta t_i - T_2|$, then the i-th coincidence event belongs to the second set $S_2$;

    1.3) Recalculate the center positions $T_1 = \Sigma_{i \in S_1} \Delta t_i$, $T_2 = \Sigma_{i \in S_2} \Delta t_i$;

    1.4) Repeat steps 1.2 and 1.3 three to five times;

    1.5) Output the center positions $S_2$, $T_2$ and the sets $S_1$, $S_2$.

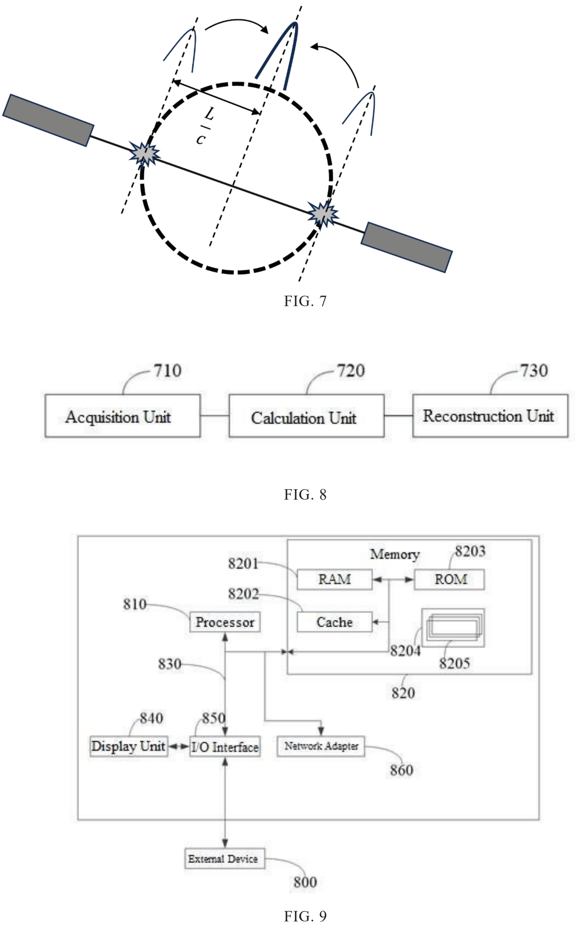

2. Let the chord length where the LOR **intersects** the shell source be L, and the speed of light be c, as shown in FIG. 7, calculating the time-of-flight difference of each coincidence event after time-of-flight compensation:

    2.1) For i = 1, 2, ..., N:

    2.1.1) If $i \in S_1$, then the compensated time difference $\Delta t_i = \Delta t_i + L/c$;

    2.1.2) If $i \in S_2$, then the compensated time difference $\Delta t_i = \Delta t_i - L/c$.

3. After the above processing, the time difference spectrum plotted with the time differences $\Delta t_i$, i = 1,2, ..., N has only one peak, as shown in for example FIG. 6.
4. Performing the above processing for all valid LORs (i.e., LORs at a distance from the central axis of the shell source that is less than the second predetermined threshold).

S230: Obtaining the TOF resolution of all the target lines of response as the prior information according to a second predetermined algorithm and the time differences of each coincidence event.
The second predetermined algorithm may be:

$$\sigma = \sqrt{\frac{\sum_{i=1}^{n}(\Delta t_i - \overline{\Delta t})^2}{n-1}}$$

where n is the total number of coincidence events on a certain target line of response, $\Delta t_i$ is the time difference of the i-th coincidence event, $\overline{\Delta t}$ is the mean value of the coincidence time differences on the target line of response, $\sigma$ is the standard deviation of the time differences on the target line of response, and the TOF resolution of the target line of response is $2.355\sigma$.
It should be particularly noted that the prior information obtained in step S230 is generally the correspondence between target lines of response and TOF resolution. In step S110, the corresponding TOF resolutions may be retrieved by indexing via lines of response identical to the target lines of response. In actual detection, all lines of response that are identical to the target lines of response may directly retrieve the corresponding TOF resolution by indexing.
Corresponding to the above image reconstruction method, the application also provides an image reconstruction system. As shown in FIG. 8, the image reconstruction system provided by the application generally comprises: an acquisition unit 710, a calculation unit 720, and a reconstruction unit 730. The acquisition unit 710 is configured to obtain TOF resolutions of a portion of lines of response according to prior information; the calculation unit 720 is configured to obtain timing resolutions of all scintillation crystals according to the TOF resolutions of the portion of lines of response and a predetermined model, and to obtain TOF resolutions of remaining lines of response according to the timing resolutions of all scintillation crystals and the predetermined model; and the reconstruction unit 730 is configured to reconstruct an image according to all TOF resolutions and a first predetermined algorithm.
The requirement for the number or proportion of the portion of lines of response is that it is sufficient to obtain the timing resolutions of all scintillation crystals according to the TOF resolutions of the portion of lines of response. It should be particularly noted that, generally speaking, an image detection device comprises a plurality of detectors. By way of example, all detectors are combined to form at least one detector ring. Each detector comprises an array consisting of a plurality of scintillation crystals and other device structures. When the connecting line between any two scintillation crystals passes through the field of view of the detector ring, it may constitute a line of response. That is, each end of a line of response is connected to a scintillation crystal. In the application, it is required that the scintillation crystals connected to the two ends of the portion of lines of response need to cover all scintillation crystals. Expressed conversely, each scintillation crystal corresponds to at least one line of response. The purpose of setting the number or proportion of the portion of lines of response in the application is to balance the amount of lines of response to be acquired and the computational load for TOF resolution calculation on the basis of ensuring that the basic data (TOF resolutions of the portion of lines of response) is sufficient to successfully obtain the TOF resolution of all lines of response. That is, the number or proportion of the portion of lines of response should be appropriate.

By way of example, the calculation unit 720 is configured to input the TOF resolutions of the portion of lines of response respectively as input values into the predetermined model to obtain the timing resolutions of all scintillation crystals, and to group the timing resolutions corresponding to two scintillation crystals located on the same line of response as one group, and input all groups of timing resolutions respectively as input values into the predetermined model to obtain the TOF resolutions of the remaining lines of response.

By way of example, the predetermined model is:

$$\sigma_{ij}^2 = \sigma_i^2 + \sigma_j^2 \qquad (1)$$

where $\sigma_i$ represents the timing resolution of scintillation crystal i, $\sigma_j$ represents the timing resolution of scintillation crystal j, and $\sigma_{ij}$ represents the TOF resolution of the line of response formed by scintillation crystal i and scintillation crystal j.

In actual operation, the timing resolutions of all scintillation crystals may be obtained by solving a system of simultaneous equations. For example, when $\sigma_{12}, \sigma_{13}, \sigma_{23}$... are known, by forming a system of simultaneous equations with the above formula (1), $\sigma_1, \sigma_2, \sigma_3$... may be obtained. By way of example, the solution of the system of simultaneous equations may adopt methods such as Preconditioned Conjugate Gradient (PCG), Minimum Residuals (MINRES), or Least Squares QR-factorization (LSQR).

Those skilled in the art should understand that, in addition to the above formula (1), the predetermined model may also be other models or algorithms, as long as the model or algorithm that may obtain the TOF resolutions of all lines of response based on the TOF resolutions of the portion of lines of response may be applied to the application. For example, in a further example of the application, the above formula (1) may be extended and expanded. In practice, the applicant has found that the TOF resolution of a line of response is not only affected by scintillation crystals, but also by the photon incident angle. Therefore, on the basis of the above formula (1), a variable $\delta_r$ may be further introduced, where r is the distance value between the line of response and the center of the field of view (FOV) on the transaxial plane of the detector ring. The photon incident angle depends on r. The relationship between r and the variable $\delta_r$ may be obtained through experience, and the variable $\delta_r$ may be indexed through r. Specifically, the above formula (1) may be transformed to obtain the following formula (2):

$$\sigma_{ij}^2 = \sigma_i^2 + \sigma_j^2 + \delta_r \qquad (2)$$

where $\sigma_i, \sigma_j, \sigma_{ij}$ have the same meaning as in the above formula (1), and $\delta_r$ represents a variable related to photon incident angle.

When obtaining the timing resolutions of scintillation crystals and the TOF resolutions of lines of response using the above formula (2) as the predetermined model, since it further associates the TOF resolutions of lines of response with the photon incident angle on the basis of associating the TOF resolutions of lines of response with scintillation crystals, it may obtain more accurate timing resolution of scintillation crystals than the above formula (1), thereby obtaining more accurate TOF resolutions of lines of response, which is more conducive to the performance of image reconstruction.

For the solution of the above formula (2), the timing resolutions of all scintillation crystals may also be obtained by solving a system of simultaneous equations. For example, when $\sigma_{ij}, \sigma_{ik}, \sigma_{jk}$... are known, by forming a system of simultaneous equations with the above formula (2), $\sigma_i, \sigma_j, \sigma_k$... may be obtained. By way of example, the solution of the system of simultaneous equations may adopt methods such as Preconditioned Conjugate Gradient (PCG), Minimum Residuals (MINRES), or Least Squares QR-factorization (LSQR). These methods may refer to the records of the prior art, and the application will not repeat them.

The first predetermined algorithm may be any algorithm that may implement image reconstruction in the field of TOF image reconstruction.

In one example, the first predetermined algorithm is, for example, a list-mode maximum-likelihood expectation-maximization (LM-MLEM) algorithm, with the specific algorithm as follows in formula (3).

$$x_j^{n+1} = \frac{x_j^n}{\sum_i H_{ij} N_i A_i} \sum_{(i,k) \in S} H_{ijk} \frac{1}{\sum_j H_{ijk} x_j^n + \frac{r_{ik}+s_{ik}}{N_i A_i}} \qquad (3)$$

where $x_j^n$ is the j-th pixel value of the n-th iteration, $N_i$ and $A_i$ are respectively the normalization factor and attenuation factor of the i-th line of response, $r_{ik}$ and $s_{ik}$ are respectively the number of random and scatter coincidence events on the k-th time bin of the i-th line of response, S is the set of line of response numbers and time bin numbers where all list-mode events are located, $H_{ij}$ is the system matrix for non-TOF reconstruction, and $H_{ijk}$ is the system matrix for TOF reconstruction.

In another example, the first predetermined algorithm is, for example, a list-mode ordered-subsets expectation-maximization (LM-OSEM) algorithm, with the specific algorithm as follows in formula (4):

$$x_j^{l+1,n} = \frac{x_j^{l,n}}{\sum_i H_{ij} N_i A_i} \sum_{(i,k) \in S_l} H_{ijk} \frac{1}{\sum_j H_{ijk} x_j^{l,n} + \frac{r_{ik}+s_{ik}}{N_i A_i}} \qquad (4)$$

where $x_j^{l,n}$ is the j-th pixel value of the l-th subset of the n-th iteration, $N_i$ and $A_i$ are respectively the normalization factor and attenuation factor of the i-th line of response, $r_{ik}$ and $s_{ik}$ are respectively the number of random and scatter coincidence events on the k-th time bin of the i-th line of response, $S_l$ is the set of line of response number i and time bin number k where all list-mode events in the l-th subset are located, $H_{ij}$ is the system matrix for non-TOF reconstruction, and $H_{ijk}$ is the system matrix for TOF reconstruction.

In another example, the first predetermined algorithm is, for example, a maximum-likelihood expectation-maximization (MLEM) algorithm, with the specific algorithm as follows in formula (5):

$$x_j^{n+1} = \frac{x_j^n}{\sum_i H_{ij} N_i A_i} \sum_i \sum_k H_{ijk} \frac{Y_{ik}}{\sum_j H_{ijk} x_j^n + \frac{r_{ik}+s_{ik}}{N_i A_i}} \qquad (5)$$

where $x_j^n$ is the j-th pixel value of the n-th iteration, $N_i$ and $A_i$ are respectively the normalization factor and attenuation factor of the i-th line of response, $r_{ik}$ and $s_{ik}$ are respectively the number of random and scatter coincidence events on the k-th time bin of the i-th line of response, $H_{ij}$ is the system matrix for non-TOF reconstruction, and $H_{ijk}$ is the system matrix for TOF reconstruction.

In another example, the first predetermined algorithm is, for example, an ordered-subsets expectation-maximization (OSEM) algorithm, with the specific algorithm as follows in formula (6):

$$x_j^{l,+1n} = \frac{x_j^{l,n}}{\sum_i H_{ij} N_i A_i} \sum_{i \in S_l} \sum_k H_{ijk} \frac{Y_{ik}}{\sum_j H_{ijk} x_j^{l,n} + \frac{r_{ik}+s_{ik}}{N_i A_i}} \qquad (6)$$

where $x_j^{l,n}$ is the j-th pixel value of the l-th subset of the n-th iteration, $N_i$ and $A_i$ are respectively the normalization factor and attenuation factor of the i-th line of response, $r_{ik}$ and $s_{ik}$ are respectively the number of random and scatter coincidence events on the k-th time bin of the i-th line of response, $S_l$ is the set of line of response number i in the l-th subset, $H_{ij}$ is the system matrix for non-TOF reconstruction, and $H_{ijk}$ is the system matrix for TOF reconstruction.

In the above examples, $H_{ijk} = H_{ij} K_{ijk}$, $K_{ijk}$ may be calculated using the following formula (7).

$$K_{ijk} = \int_{T_0}^{T_1} \frac{1}{\sqrt{2\pi}\sigma} \exp\left(-\frac{t^2}{2\sigma^2}\right) dt \qquad (7)$$

where T0 is the start time of the k-th time bin, T1 is the end time of the k-th time bin, t is an integration variable, and σ may adopt the data obtained in the predetermined model in the above step S130.

Compared with the prior art, the image reconstruction system provided by the application applies differentiated TOF resolutions to different lines of response. In particular, by correlating the TOF resolutions of lines of response with the timing resolutions of corresponding scintillation crystals, the TOF resolutions of lines of response may be accurately estimated and applied to TOF image reconstruction, thereby effectively enhancing image reconstruction quality.

Regarding prior information, reference may be made to the description in the above image reconstruction method, which will not be repeatedly elaborated here.

In some embodiments, an electronic device is provided, which may include a processor and a memory storing a computer program, wherein the processor is configured to execute the method according to any embodiment of the application when running the computer program.

As shown in FIG. 9, the electronic device is presented in the form of a general computing device. The components of the electronic device may comprise but are not limited to: at least one processor 810, at least one memory 820, a bus 830 connecting different system components (including the memory 820 and the processor 810), a display unit 840, etc. The memory 820 stores program code, which may be executed by the processor 810, such that the processor 810 executes the methods according to various exemplary embodiments of the application described in this specification. For example, the processor 810 may execute the methods shown in FIGS. 1-2.

The memory 820 may include a readable medium in the form of volatile storage units, such as a random access storage unit (RAM) 8201 and/or a cache storage unit 8202, and may further include a read-only storage unit (ROM) 8203.

The memory 820 may also include a program/utility 8204 having a set (at least one) of program modules 8205. Such program modules 8205 include but are not limited to: an operating system, one or more application programs, other program modules, and program data. Each of these examples or some combination thereof may include an implementation of a network environment.

The bus 830 may represent one or more of several types of bus structures, including a storage unit bus or storage unit controller, a peripheral bus, a graphics acceleration port, a processor, or a local bus using any of a variety of bus structures.

The electronic device may also communicate with one or more external devices 800 (such as a keyboard, a pointing device, a Bluetooth device, etc.), may also communicate with one or more devices that enable a user to interact with the electronic device, and/or communicate with any devices (such as a router, a modem, etc.) that enable the electronic device to communicate with one or more other computing devices. Such communication may be performed through an input/output (I/O) interface 850. Moreover, the electronic device may also communicate with one or more networks (such as a local area network (LAN), a wide area network (WAN), and/or a public network, such as the Internet) through a network adapter 860. The network adapter 860 may communicate with other modules of the electronic device through the bus 830. It should be understood that although not shown in the figure, other hardware and/or software modules may be used in conjunction with the electronic device, including but not limited to: microcode, device drivers, redundant processing units, external disk drive arrays, RAID systems, tape drives, and data backup storage systems, etc.

Through the description of the above embodiments, those skilled in the art will readily understand that the exemplary embodiments described herein may be implemented by software, or may be implemented by combining software with necessary hardware. The technical solution according to the embodiments of the application may be embodied in the form of a software product, which may be stored in a computer-readable storage medium (which may be a CD-ROM, a U disk, a mobile hard disk, etc.) or on a network, including computer program instructions that cause a computing device (which may be a personal computer, a server, or a network device, etc.) to execute the above method according to the embodiments of the application.

The software product may employ any combination of one or more readable media. The readable medium may be a readable signal medium or a readable storage medium. The readable storage medium may be, for example, but not limited to, an electrical, magnetic, optical, electromagnetic, infrared, or semiconductor system, apparatus, or device, or any combination of the above. More specific examples (a non-exhaustive list) of the readable storage medium include: an electrical connection having one or more wires, a portable disk, a hard disk, a random access memory (RAM), a read-only memory (ROM), an erasable programmable read-only memory (EPROM or flash memory), an optical fiber, a portable compact disk read-only memory (CD-ROM), an optical storage device, a magnetic storage device, or any suitable combination of the above.

The computer-readable storage medium may include a data signal propagated in baseband or as part of a carrier wave, in which readable program code is carried. Such a propagated data signal may take various forms, including but not limited to electromagnetic signals, optical signals, or any suitable combination of the above. The readable storage medium may also be any readable medium other than a readable storage medium, and the readable medium may send, propagate, or transmit a program for use by or in connection with an instruction execution system, apparatus, or device. The program code contained on the readable storage medium may be transmitted using any appropriate medium, including but not limited to wireless, wired, optical cable, RF, etc., or any suitable combination of the above.

Program code for executing the operations of the application may be written in any combination of one or more programming languages. The programming languages include object-oriented programming languages-such as Java, C++, etc., and also include conventional procedural programming languages-such as C language or similar programming languages. The program code may execute entirely on a user computing device, partly on a user device, as a stand-alone software package, partly on a user computing device and partly on a remote computing device, or entirely on a remote computing device or server. In the case of involving a remote computing device, the remote computing device may be connected to the user computing device through any kind of network, including a local area network (LAN) or a wide area network (WAN), or may be connected to an external computing device (for example, connected through the Internet using an Internet service provider).

The above-mentioned computer-readable medium carries one or more program instructions, and when the above one or more program instructions are executed by a device, the computer-readable medium implements the aforementioned functions.

Through the description of the above embodiments, those skilled in the art may readily understand that the exemplary embodiments described herein may be implemented by software, or may be implemented by combining software with necessary hardware. The technical solution according to the embodiments of the application may be embodied in the form of a software product, which may be stored in a computer-readable storage medium (which may be a CD-ROM, a U disk, a mobile hard disk, etc.) or on a network, including computer program instructions that cause a computing device (which may be a personal computer, a server, or a network device, etc.) to execute the above method according to the embodiments of the application.

Those skilled in the art may understand that the above modules may be distributed in the apparatus according to the description of the embodiments, or may be changed and located in one or more apparatuses other than that of the present embodiment. The modules of the above embodiments may be combined into one module, or may be further split into multiple sub-modules.

Although the application provides method operation steps as described in the above embodiments or flowcharts, based on routine or non-inventive efforts, more or fewer operation steps may be included in the method. For steps that do not have a necessary causal relationship in logic, the execution order of these steps is not limited to the execution order provided by the embodiments of the application.

The various embodiments in this specification are described in a progressive manner, and the same or similar parts between the various embodiments may be referred to each other. Each embodiment focuses on the differences from other embodiments.

The foregoing provides a detailed description of the embodiments of the application. Specific examples have been used herein to illustrate the principles and implementations of the application. The description of the foregoing embodiments is intended only to facilitate understanding of the method and core idea of the application. At the same time, for those skilled in the art, changes or modifications to the specific implementations and application scope based on the ideas of the application fall within the scope of protection of the application. In summary, the content of this specification should not be construed as limiting the application.

**Claims**

1.  An image reconstruction method, **characterized by** comprising:

    obtaining TOF resolutions of a portion of lines of response according to prior information;
    obtaining timing resolutions of all scintillation crystals according to the TOF resolutions of the portion of lines of response and a predetermined model;
    obtaining TOF resolutions of remaining lines of response according to the timing resolutions of all scintillation crystals and the predetermined model; and
    reconstructing an image according to all TOF resolutions and a first predetermined algorithm.

2.  The image reconstruction method according to claim 1, **characterized in that** the obtaining timing resolutions of all scintillation crystals according to the TOF resolutions of the portion of lines of response and a predetermined model comprises:

    inputting the TOF resolutions of the portion of lines of response as input values respectively into the predetermined model to obtain the timing resolutions of all scintillation crystals; and
    the obtaining TOF resolutions of remaining lines of response according to the timing resolutions of all scintillation crystals and the predetermined model comprises:
    grouping the timing resolutions corresponding to two scintillation crystals located on the same line of response as one group, and inputting all groups of timing resolutions as input values respectively into the predetermined model to obtain the TOF resolutions of the remaining lines of response.

3. The image reconstruction method according to claim 1, **characterized in that** the predetermined model is:

$$\sigma_{ij}^2 = \sigma_i^2 + \sigma_j^2$$

where $\sigma_i$ represents the timing resolution of scintillation crystal i, $\sigma_j$ represents the timing resolution of scintillation crystal j, and $\sigma_{ij}$ represents the TOF resolution of the line of response formed by scintillation crystal i and scintillation crystal j.

4. The image reconstruction method according to claim 1, **characterized in that** the predetermined model is:

$$\sigma_{ij}^2 = \sigma_i^2 + \sigma_j^2 + \delta_r$$

where $\sigma_i$ represents the timing resolution of scintillation crystal i, $\sigma_i$ represents the timing resolution of scintillation crystal j, $\delta_r$ represents a variable related to photon incident angle, and $\sigma_{ij}$ represents the TOF resolution of the line of response formed by scintillation crystal i and scintillation crystal j.

5. The image reconstruction method according to claim 1, **characterized in that** the prior information is obtained according to the following steps:

conducting an image detection test under test conditions to obtain the number of coincidence events on all lines of response and time difference corresponding to two crystals of each coincidence event ;
selecting lines of response where the number of coincidence events reaches a first predetermined threshold as target lines of response; and
obtaining the TOF resolution of all the target lines of response as the prior information according to a second predetermined algorithm and the time difference of each coincidence event.

6. The image reconstruction method according to claim 5, **characterized in that** the second predetermined algorithm is:

$$\sigma = \sqrt{\frac{\sum_{i=1}^n (\Delta t_i - \overline{\Delta t})^2}{n-1}}$$

where n is the total number of coincidence events on a certain target line of response, $\Delta t_i$ is the time difference of the i-th coincidence event, $\overline{\Delta t}$ is the mean value of the coincidence time differences on the target line of response, $\sigma$ is the standard deviation of the time differences on the target line of response, and the TOF resolution of the target line of response is $2.355\sigma$.

7. The image reconstruction method according to claim 5, **characterized in that** the first predetermined threshold is greater than or equal to 5.

8. The image reconstruction method according to claim 5, **characterized in that** the test conditions comprise:

a radioactive source that only emits positrons being selected as a target source; and/or
the shape of the target source comprising a shell source, a line source, or a point source.

9. The image reconstruction method according to claim 8, **characterized in that** when the target source is the shell source, before selecting lines of response where the number of coincidence events exceeds the first predetermined threshold as target lines of response, the steps for obtaining the prior information further comprise:
excluding lines of response at a distance from the central axis of the shell source that reaches a second predetermined threshold.

10. The image reconstruction method according to claim 9, **characterized in that** the second predetermined threshold is 80% of the maximum distance corresponding to the field of view of the detector ring.

11. The image reconstruction method according to claim 8, **characterized in that** when the target source is the shell source, after selecting lines of response where the number of coincidence events exceeds the first predetermined threshold as target lines of response, the steps for obtaining the prior information further comprise:
performing time-of-flight compensation on all coincidence events using a predetermined method.

12. The image reconstruction method according to claim 11, **characterized in that** the predetermined method comprises:

obtaining two intersection points between the line of response and the shell source, and obtaining the time-of-flight difference of coincidence events generated by annihilation at the two intersection points;
plotting a time difference spectrum according to the time differences of the coincidence events to obtain two peaks, where the two peaks respectively correspond to the two intersection points between the line of response and the shell source;
classifying the coincidence events into one of the two peaks using a k-means algorithm; and
compensating the time-of-flight differences of the coincidence events according to the intersection point to which the respective coincidence events correspond and the calculated time-of-flight difference.

13. The image reconstruction method according to claim 8, **characterized in that** the test conditions further comprise:
the size of the target source being less than a predetermined requirement.

14. The image reconstruction method according to claim 13, **characterized in that** the predetermined requirement comprises:

the thickness of the shell source being less than or equal to 1 cm; or
the diameter of the line source being less than or equal to 1 cm; or
the diameter of the point source being less than or equal to 0.5 cm.

15. The image reconstruction method according to claim 8, **characterized in that** the test conditions further comprise:
the position of the target source ensuring that the connecting lines between any single detection unit and at least two detection units at different positions all pass through the target source.

16. The image reconstruction method according to claim 15, **characterized in that**:

the height extent of the shell source is parallel to the axis of the PET; or
among the intersection points between the cross-section of the detector ring and the line source, at least three points are not collinear; or
at least four of the point sources are not coplanar.

17. The image reconstruction method according to claim 8, **characterized in that** the target source comprises Fluorine-18 ($^{18}$F), Sodium-22 ($^{22}$Na), or Germanium-68 ($^{63}$Ge).

18. The image reconstruction method according to claim 1, **characterized in that** the first predetermined algorithm is:

$$x_j^{n+1} = \frac{x_j^n}{\sum_i H_{ij} N_i A_i} \sum_{(i,k) \in S} H_{ijk} \frac{1}{\sum_j H_{ijk} x_j^n + \frac{r_{ik} + s_{ik}}{N_i A_i}}$$

where $x_j^n$ is the j-th pixel value of the n-th iteration, $N_i$ and $A_i$ are respectively the normalization factor and attenuation factor of the i-th line of response, $r_{ik}$ and $s_j$. are respectively the number of random and scatter coincidence events on the k-th time bin of the i-th line of response, S is the set of line of response numbers and time bin numbers where all list-mode events are located, $H_{ij}$ is the system matrix for non-TOF reconstruction, and $H_{ijk}$ is the system matrix for TOF reconstruction.

19. The image reconstruction method according to claim 1, **characterized in that** the first predetermined algorithm is:

$$x_j^{l+1,n} = \frac{x_j^{l,n}}{\sum_i H_{ij} N_i A_i} \sum_{(i,k)\in S_l} H_{ijk} \frac{1}{\sum_j H_{ijk} x_j^{l,n} + \frac{r_{ik}+s_{ik}}{N_i A_i}}$$

where $x_j^{l,n}$ is the j-th pixel value of the l-th subset of the n-th iteration, $N_i$ and $A_i$ are respectively the normalization factor and attenuation factor of the i-th line of response, $r_{ik}$ and $s_{ik}$ are respectively the number of random and scatter coincidence events on the k-th time bin of the i-th line of response, $S_l$ is the set of line of response number i and time bin number k where all list-mode events in the l-th subset are located, $H_{ij}$ is the system matrix for non-TOF reconstruction, and $H_{ijk}$ is the system matrix for TOF reconstruction.

20. The image reconstruction method according to claim 1, **characterized in that** the first predetermined algorithm is:

$$x_j^{n+1} = \frac{x_j^n}{\sum_i H_{ij} N_i A_i} \sum_i \sum_k H_{ijk} \frac{Y_{ik}}{\sum_j H_{ijk} x_j^n + \frac{r_{ik}+s_{ik}}{N_i A_i}}$$

where $x_j^n$ is the j-th pixel value of the n-th iteration, $N_i$ and $A_i$ are respectively the normalization factor and attenuation factor of the i-th line of response, $r_{ik}$ and $s_{ik}$ are respectively the number of random and scatter coincidence events on the k-th time bin of the i-th line of response, $H_{ij}$ is the system matrix for non-TOF reconstruction, and $H_{ijk}$ is the system matrix for TOF reconstruction.

21. The image reconstruction method according to claim 1, **characterized in that** the first predetermined algorithm is:

$$x_j^{l,+1n} = \frac{x_j^{l,n}}{\sum_i H_{ij} N_i A_i} \sum_{i\in S_l} \sum_k H_{ijk} \frac{Y_{ik}}{\sum_j H_{ijk} x_j^{l,n} + \frac{r_{ik}+s_{ik}}{N_i A_i}}$$

where $x_j^{l,n}$ is the j-th pixel value of the l-th subset of the n-th iteration, $N_i$ and $A_i$ are respectively the normalization factor and attenuation factor of the i-th line of response, $r_{ik}$ and $s_{ik}$ are respectively the number of random and scatter coincidence events on the k-th time bin of the i-th line of response, $S_l$ is the set of line of response number i in the l-th subset, $H_{ij}$ is the system matrix for non-TOF reconstruction, and $H_{ijk}$ is the system matrix for TOF reconstruction.

22. The image reconstruction method according to any one of claims 18 to 21, **characterized in that**:

$$H_{ijk} = H_{ij} K_{ijk}$$
$$K_{ijk} = \int_{T_0}^{T_1} \frac{1}{\sqrt{2\pi}\sigma} \exp\left(-\frac{t^2}{2\sigma^2}\right) dt$$

where T0 is the start time of the k-th time bin, T1 is the end time of the k-th time bin, t is an integration variable, and $\sigma$ represents the TOF resolution of the line of response.

23. An image reconstruction system, **characterized in that** it comprises:

an acquisition unit, configured to obtain TOF resolutions of a portion of lines of response according to prior information;
a calculation unit, configured to obtain timing resolutions of all scintillation crystals according to the TOF resolutions of the portion of lines of response and a predetermined model, and to obtain TOF resolutions of remaining lines of response according to the timing resolutions of all scintillation crystals and the predetermined model; and
a reconstruction unit, configured to reconstruct an image according to all TOF resolutions and a first predetermined algorithm.

**24.** The image reconstruction system according to claim 23, **characterized in that** the calculation unit is configured to input the TOF resolutions of the portion of lines of response as input values respectively into the predetermined model to obtain the timing resolutions of all scintillation crystals, and to group the timing resolutions corresponding to two scintillation crystals located on the same line of response as one group, and input all groups of timing resolutions as input values respectively into the predetermined model to obtain the TOF resolutions of the remaining lines of response.

**25.** A detection device, **characterized in that** it comprises the image reconstruction system according to claim 23 or 24.

**26.** An electronic device, **characterized by** comprising: a processor and a memory storing a computer program, wherein the processor is configured to execute the computer program to implement the method according to any one of claims 1 to 22.

**27.** A storage medium, **characterized in that** the storage medium stores a computer program that, when executed, implements the method according to any one of claims 1 to 22.

Obtaining TOF resolutions of a portion of lines of response according to prior information — S110

Obtaining timing resolutions of all scintillation crystals according to the TOF resolutions of the portion of lines of response and a predetermined model — S120

Obtaining TOF resolutions of the remaining lines of response according to the timing resolutions of all scintillation crystals and the predetermined model — S130

Reconstructing an image according to all TOF resolutions and a first predetermined algorithm — S140

FIG. 1

Conducting an image detection test under test conditions to obtain the number of coincidence events on all lines of response and the time difference corresponding to the two crystals in each coincidence event — S210

Selecting lines of response where the number of coincidence events reaches a first predetermined threshold as target lines of response — S220

Obtaining the TOF resolutions of all the target lines of response as the prior information according to a second predetermined algorithm and the time difference in each coincidence event — S230

FIG. 2

EP 4 737 949 A1

FIG. 3

FIG. 4

FIG. 5

FIG. 6

FIG. 7

FIG. 8

FIG. 9

## INTERNATIONAL SEARCH REPORT

| International application No. |
| --- |
| **PCT/CN2024/135750** |

### A. CLASSIFICATION OF SUBJECT MATTER

G01T1/161(2006.01)i;  G06T11/00(2006.01)i

According to International Patent Classification (IPC) or to both national classification and IPC

### B. FIELDS SEARCHED

Minimum documentation searched (classification system followed by classification symbols)

IPC: G01T1 G06T11

Documentation searched other than minimum documentation to the extent that such documents are included in the fields searched

Electronic data base consulted during the international search (name of data base and, where practicable, search terms used)

CNABS, CNTXT, CNKI: 响应线, 轨迹线, 飞行时间, 时间, 分辨率, 分辨力, 闪烁体, 晶体, 探测器, 检测器, 角度, 经验, 先验, 前验, 符合: VEN, USTXT, EPTXT, WOTXT, IEEE: LOR, line of response, TOF, time of flight, time, temporal, resolution, scintillator, crystal, detector, angle, prior, coincidence.

### C. DOCUMENTS CONSIDERED TO BE RELEVANT

| Category* | Citation of document, with indication, where appropriate, of the relevant passages | Relevant to claim No. |
| --- | --- | --- |
| PX | CN 117653169 A (HEFEI RUISHI DIGITAL TECHNOLOGY CO., LTD.) 08 March 2024 (2024-03-08)<br>  description, paragraphs 76-195, and figures 1-9 | 1-27 |
| PX | CN 117653168 A (HEFEI RUISHI DIGITAL TECHNOLOGY CO., LTD.) 08 March 2024 (2024-03-08)<br>  description, paragraphs 79-199, and figures 1-9 | 1-27 |
| A | CN 112998735 A (ZHONGPAI S&T (SHENZHEN) CO., LTD. et al.) 22 June 2021 (2021-06-22)<br>  description, paragraphs 67-149, and figures 1-11 | 1-27 |
| A | CN 107978002 A (SHANGHAI NEUSOFT MEDICAL TECHNOLOGIES, LTD.) 01 May 2018 (2018-05-01)<br>  entire document | 1-27 |
| A | US 2019287275 A1 (KONINKLIJKE PHILIPS N.V.) 19 September 2019 (2019-09-19)<br>  entire document | 1-27 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **19 February 2025** | **21 February 2025** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/135750** |

| C. | DOCUMENTS CONSIDERED TO BE RELEVANT | |
| --- | --- | --- |
| Category* | Citation of document, with indication, where appropriate, of the relevant passages | Relevant to claim No. |
| A | 李俊 (LI, Jun). "TOF-PET 图像快速重建算法及其在稀疏探测器系统上的应用 (Efficient TOF-PET Image Reconstruction Algorithm and Its Application on Sparse Detectors)" 中国优秀硕士学位论文全文数据库医药卫生科技辑 (*China Master's Theses Full-text Database, Medicine and Health Sciences*), No. 01, 15 January 2022 (2022-01-15), entire document | 1-27 |
| A | WADHWA, P. et al. "PET Image Reconstruction Using Physical and Mathematical Modelling For Time of Flight PET-MR Scanners in the STIR Library" *Methods*, 29 January 2020 (2020-01-29), entire document | 1-27 |

Form PCT/ISA/210 (second sheet) (July 2022)

**INTERNATIONAL SEARCH REPORT**
**Information on patent family members**

International application No.

**PCT/CN2024/135750**

| Patent document cited in search report | | | Publication date (day/month/year) | Patent family member(s) | | | Publication date (day/month/year) |
|---|---|---|---|---|---|---|---|
| CN | 117653169 | A | 08 March 2024 | None | | | |
| CN | 117653168 | A | 08 March 2024 | None | | | |
| CN | 112998735 | A | 22 June 2021 | CN | 112998735 | B | 02 September 2022 |
| CN | 107978002 | A | 01 May 2018 | US | 2018114346 | A1 | 26 April 2018 |
| | | | | US | 10319118 | B2 | 11 June 2019 |
| | | | | CN | 107978002 | B | 05 January 2021 |
| US | 2019287275 | A1 | 19 September 2019 | US | 10977841 | B2 | 13 April 2021 |
| | | | | EP | 3494547 | A1 | 12 June 2019 |
| | | | | EP | 3494547 | B1 | 17 June 2020 |
| | | | | WO | 2018024487 | A1 | 08 February 2018 |
| | | | | JP | 2019525179 | A | 05 September 2019 |
| | | | | JP | 7118048 | B2 | 15 August 2022 |
| | | | | CN | 109564692 | A | 02 April 2019 |
| | | | | CN | 109564692 | B | 14 November 2023 |

Form PCT/ISA/210 (patent family annex) (July 2022)

**REFERENCES CITED IN THE DESCRIPTION**

**Patent documents cited in the description**

- CN 202311859527